# EUROPEAN PATENT SPECIFICATION

(11) **EP 3 448 148 B1**
(45) Date of publication and mention of the grant of the patent: **26.05.2021**
(21) Application number: 16721231.5
(22) Date of filing: 31.03.2016
(51) Int. Cl.: A01K 67/027, C12N 5/0735, G01N 33/50

(54) **INDICATOR STEM CELL LINE/LIVING ORGANISM (NON-HUMAN) AND A METHOD FOR DETECTION OF THE GENOTOXIC POTENTIAL OF AQUATIC SAMPLES OR AQUEOUS SOLUTIONS OF TEST COMPOUNDS**
INDIKATORSTAMMZELLLINIE/LEBENDER ORGANISMUS (NICHT-HUMAN) UND VERFAHREN ZUM NACHWEIS DES GENOTOXISCHEN POTENZIALS VON WASSERPROBEN ODER WÄSSRIGEN LÖSUNGEN VON TESTVERBINDUNGEN
LIGNÉE DE CELLULES SOUCHES/ORGANISME VIVANT (NON HUMAIN) D'INDICATION ET PROCÉDÉ DE DÉTECTION DU POTENTIEL GÉNOTOXIQUE D'ÉCHANTILLONS AQUATIQUES OU DE SOLUTIONS AQUEUSES DE COMPOSÉS DE TEST

(43) Date of publication of application: 06.03.2019
(73) Proprietor: Gobio GmbH Institut für Gewässerökologie und angewandte Biologie, 65326 Aarbergen/Kettenbach (DE)
(72) Inventor: HESCHELER, Jürgen, 50968 Köln (DE); PFANNKUCHE, Kurt, 53359 Rheinbach (DE); STAHLSCHMIDT-ALLNER, Petra, 65326 Aarbergen / Kettenbach (DE)
(74) Representative: Mackert, Andreas
(86) International application number: PCT/IB2016/051822
(87) International publication number: WO 2017/168210

(56) References cited:
- US-A- 5 229 265
- US-A1- 2008 138 820
- PAULS S ET AL: "A zebrafish histone variant H2A.F/Z and a transgenic H2A.F/Z:GFP fusion protein for in vivo studies of embryonic development", DEVELOPMENT, GENES AND EVOLUTION, BERLIN, DE, vol. 211, 1 January 2001 (2001-01-01), pages 603-610, XP002971408, ISSN: 0949-944X, DOI: 10.1007/S00427-001-0196-X
- ADLEN FOUDI ET AL: "Analysis of histone 2B-GFP retention reveals slowly cycling hematopoietic stem cells", NATURE BIOTECHNOLOGY, vol. 27, no. 1, 1 January 2009 (2009-01-01), pages 84-90, XP055320764, US ISSN: 1087-0156, DOI: 10.1038/nbt.1517
- K. R. HUGHES ET AL: "Heterogeneity in histone 2B-green fluorescent protein-retaining putative small intestinal stem cells at cell position 4 and their absence in the colon", AMERICAN JOURNAL OF PHYSIOLOGY - GASTROINTESTINAL AND LIVER PHYSIOLOGY, vol. 303, no. 11, 20 September 2012 (2012-09-20), pages G1188-G1201, XP055320775, ISSN: 0193-1857, DOI: 10.1152/ajpgi.00080.2012
- KANDA TERU ET AL: "Histone-GFP fusion protein enables sensitive analysis of chromosome dynamics in living mammalian cells", CURRENT BIOLOGY, CURRENT SCIENCE, GB, vol. 8, no. 7, 26 March 1998 (1998-03-26), pages 377-385, XP002176075, ISSN: 0960-9822, DOI: 10.1016/S0960-9822(98)70156-3
- GUSTAVINO B.; SCORNAJENGHI K. A.; MINISSI S.; CICCOTTI E.: "Micronuclei induced in erythrocytes of Cyprinus carpio (teleostei, pisces) by X-rays and colchicine", MUTAT RES., vol. 494, no. 1-2, 2001, pages 151-159, XP055320660, cited in the application
- AYLON F.; GARCIA-VASQUEZ, E.: "Induction of micronuclei and other nuclear abnormalities in European minnow Phoxinus phoxinus and mollie Poecilia latipinna: an assessment of the fish micronucleus test", MUTAT RES., vol. 467, no. 2, 2000, pages 177-186, XP0027400542, cited in the application

## Description

### Summary of the Invention

This invention presents a reporter gene technology for non-destructive, self-signalizing visualisation of nuclear structures, which allows the identification of nuclear and chromosome anomalies - including micronuclei - as consequence of exposure to genotoxic compounds. This technology for visu-alising micronuclei can be applied in in vitro as well as in vivo toxicological models which are transgenic for histone associated fluorescent protein. Furthermore, a corresponding non-human transgenic stem cell line based method for the detection of the genotoxic potential of aquatic samples or aqueous solutions of test compounds is presented. This cell line has been employed in the development of kits suitable for the detection of the genotoxic potential of samples by the inventive method.

### Background

The micronucleus test is a toxicological assay to assess the genotoxic potential of substances based on the formation of micronuclei (MN). It is recognized as one of the most important tools for assessing the genotoxicity of environmental samples and newly developed chemicals, and is therefore recommended by the OECD for drug screening and ecotoxicological assessment of genotoxic potential.

### State of the art

The formation of micronuclei may be induced either by deterring the migration of whole chromosomes to the cell poles during anaphase (aneugens) or by promoting the formation of acentric chromosomes (clastogens).

Micronuclei represent damages transmitted to daughter cells after mitosis, hence assessing somatic inherited genetic damage that may not be assessed in the traditional chromosome aberrations test scored in metaphase cells (OECD TG 473). Thus, assessment of MN has become a mandatory criterion in environmental risk assessment and drug screening. Two major versions of the test have become standards in toxicological testing, the *in vivo* mammalian erythrocyte micronucleus test (OECD TG 474) and the *in vitro* mammalian cell micronucleus test (OECD TG 487).

In spite of its importance due to metabolic and pharmacokinetic factors difficult to reproduce in *in vitro* systems, the *in vivo* mammalian erythrocyte micronucleus test presents limitations that may only be overcome with *in vitro* testing. Many compounds (for instance topically applied or inhaled pharmaceuticals) are not systemically absorbed in living organisms, so that bioavailability for the target tissue (in this case, the bone marrow) may be low or negligible. Moreover, inter-individual variability in spontaneous MN frequency poses a severe difficulty for the *in vivo* system, demanding a thorough identification of basal MN rates for valid results to be obtained. Besides, the increasing ethical considerations regarding animal testing with vertebrates, and public and political pressure for the development of *in vitro* alternatives in toxicological assays, favour the usage of cell culture-based systems for the identification of the genotoxic potential of samples.

The *in vitro* micronucleus test (MNvit) basically consists of exposing cultured cells to a dilution series of a substance that potentially induces MN formation and comparing MN frequency with positive (i.e. aneugens and clastogens) and negative (i.e. solvent) controls after cell division. MN frequency is estimated by visually scoring micronuclei in each test group and the controls after nuclei-specific staining (e.g. Giemsa, acridine orange, propidium iodide) and slide preparation. Asides from initial culturing of cells (including extraction from the donor organism in the case of primary cells), this time-consuming process encompasses exposure, harvesting, staining and scoring. An automatic scoring technique of MN frequency by flow cytometry has been validated, but the method is cost- and knowledge-intensive, requiring excellence in both personnel and laboratory facilities in harvesting, staining and scoring of cells.

Different techniques may be used in cell harvesting and slide preparation, as long as high-quality cell preparations for scoring are obtained and cytoplasm is retained for MN assignment. To date, validity of the MNvit assay using various rodent cell lines (CHO, V79, CHL/IU, and L5178Y) and human lymphocytes has been demonstrated, but generally cell types (and/or lines) with a low, stable background MN frequency are recommended for the assay. In cell lines specifically, background MN frequency has been shown to vary from around 0.3 - 2 % (CHL/IU, V79) up to approximately 4.7 % (in the human HeLA cell line).

Slide preparation for visual scoring requires fixation of harvested cells, so that post-exposure evaluation reflects established effects only. Although some methods employing (supra)vital staining have been established for flow cytometry scoring, survival of cells after sorting is usually limited and does not allow for posterior monitoring of cell status and proliferation, hence implying that post-exposure data on cell survival rates, promotion of neoplasia in cultured cells and other related information could not be assessed until now.

In the article of Pauls, S. et al: "A zebrafish histone variant H2A.F/Z and a transgenic H2A.F/Z:GFP fusion protein for in vivo studies of embryonic development", DEVELOPMENT, GENES AND EVOLUTION, BERLIN, DE, vol.211, 1 January 2001 (2001-01-01), pages 603-610, XP002971408, ISSN: 0949-944X, DOI: 10.1007/S00427-0196-X, a transgenic zebrafish expressing a H2A/F/Z::GFP fusion protein capable of identifying nuclear and chromosomal anomalies is disclosed, but no indication is given, that said fusion protein allows an indentification of micronuclei as well. Furthermore, the GFP-mediated fluorescence is visible in all cells of the body of the transgenic fish at all embryonic and larval stages, so the scoring leads to unclear results, because the nuclei of all cells in the body of the transgenic fish embryones or larval stages send a visible signal, even overlaying cells of different cellular tissues and cellular types.

Adlen Foudi et al. in their article "Analysis of histone 2B-green retention reveals slowly cycling hemtopoietic stem cells", NATURE BIOTECHNOLOGY, vol. 27, no. 1 January 2009 (2009-01-01), pages 84-90, XP055320764, US, ISSN: 1087-0156, DOI: 10.1038/nbt. 1517, as well as K. R. Hughes et al. in their article "Heterogeneity in histone 2B-green fluorescence protein-retaining putative small intestinal stem cells at cell poition 4 and their absence in the colon", AMERICAN JOURNAL OF PHYSIOLOGY, vol. 303, no. 11, 20 September 2012 (2012-09-20), pages G 1188- G 1201, XP055320775, ISSN: 0193-1857, DOI: 10.1152/ajpgi.00080.2012, refer to transgenic organism in which expression of H2BGFP is triggered by exogenous cues (e.g. doxycycline supplied by drinking water), resulting in GFP label of cells that divided after triggering GFP expression.

Given that GFP expression is triggered for delimited time span this approach enables recording of cell cycle data, but is not suited for toxicological purposes due to i) chemical exposure causing gene expression, ii) disadvantage of non-selective labelling of all cells and iii) absence of nuclei in mammalian erythrocytes.

The US 2008/138820 A1 (Thomas Nicholas (GB)) 12 June 2008 (2008-06-12)presents a technique which is not based on i) H2B transgenic cells, ii) is not non-destructive, that means accessible to live imagining high throughput technologies, iii) the core endpoint nuclear structures and micronuclei are not self-signalizing, that means destructive staining after fixation is required. Given that the mammalian cells which are declared as suitable for the technologies are immortalized and the destructive detection methods applied, the technology presented in the US 2008/138820 A1 is not suited for cancerogenic transformation monitoring in downstream analyses of genotoxin exposed cells.

Kanda Teru et al. mention in their article: "Histone-GFP fusion protein enables sensitive analysis of chromosome dynamics in living mammalian cells", CURRENT BIOLOGY, CURRENT SCIENCE, GB, vol. 8, no. 7, 26 March 1998 (1998-03-26), pages 377-385, XP002176075, ISSN: 0960-9822, DOI: 10.1016/S0960-9822(98)70156-3, Histone::GFP fusion proteins only in the context of analyzing chromosome dynamics in living cells without referring to micronuclei.

Thus, a method for scoring MN in living cells that may be observed after scoring would enable the investigation of long-lasting effects of genotoxic compounds, and the provision of easy-to-handle kits containing cells with fluorescence-labelled nuclei suitable for the assessment of genotoxic potential - without need for harvesting or staining of cells - would greatly simplify routine laboratory work in genotoxic survey.

This invention presents a non-human indicator stem cell line as well as a non-human indicator living organism for non-destructive, self-signalizing visualisation of nuclear structures, which allows the identification of nuclear and chromosome anomalies as consequence of exposure to genotoxic compounds.

The inventive new stem cell line is from the Koi carp (*Cyprinus carpio haematopterus*) brain (KCB) with nuclei successfully labelled with green fluorescent protein fused to the histone 2B (H2B-GFP), thus presenting enormous potential for assessing the cytogenetic status of cells. A background MN frequency around 0.6 % and the ability to induce micronuclei in this cell line with both aneugens and clastogens has been successfully demonstrated in a series of preliminary experiments (see below), allowing for the development of kits of cells with fluorescence-labelled nuclei ready for sample exposure and scoring of nuclei / micronuclei.

Furthermore, this invention also presents a living non-human organism, which is a transgenic fish, namely medaka (*Oryzias latipes*), as a living indicator organism with cell lineages (such as germ cells or erythroid cells) showing a lineage specific histone associated fluorescent protein expression. MN visualisation is possible in translucent embryos or by ex vivo preparation of blood smears.

### Material and methods

This new and inventive method for genotoxic potential assessment of aquatic samples or aqueous solutions employs an innovative transgenic stem cell line of non-human animal origin and/or a cell lineage (of the erythrocytic series) of a transgenic non-human animal (in this case the medaka, *Oryzias latipes*) for the visualisation of nuclear structures. In this cell line and/or cell lineage, a fluorescent protein is fused to a histone in order to restrict the signal to nuclei, preferentially to the histone 2B. The initial fluorescent protein reporter of choice is the enhanced green fluorescent protein (eGFP), but other GFP derivatives (e.g. YFP, EBFP) as well as derivatives from other fluorescent proteins such as DsRed (e.g. mRFP, mCherry) may be employed. The transgenic stem cells are pluripotent and tissue-regenerating, and are preferably derived from vertebrates, especially from fish. The donor organism used in the first cell line prototype was the Koi carp (*Cyprinus carpio haematopterus*), and for this first assembly stem cells were obtained from the brain. The first transgenic fish prototype employed in this method was the Japanese rice fish or medaka (*Oryzias latipes*) in which a cell lineage e.g. the erythrocytic series has nuclei labelled with a histone-associated fluorescent protein.

The stem cell line employed in the original method described herein is deposited, in accordance with the Budapest Treaty, at the "Deutsche Sammlung von Mikroorganismen und Zellkulturen (DSMZ)", under the number DSM ACC3285.

The presented new method for the detection of the genotoxic potential of aquatic samples and/or aqueous solutions of test compounds - by using a transgenic stem cell line from a non-human animal and/or a cell lineage of a non-human animal with nuclei labelled with fluorescent protein fused to a chromatin-associated protein - comprises cell lines with fluorescence-labelled nuclei growing as a monolayer on cell culture plates with cell-culture media to be supplemented with different concentrations of the potentially genotoxic test compounds, pipetting these media to the exposure wells on at least one cell culture plate and comparing micronucleus frequencies with positive and negative controls after cell division, by non-destructive visually scoring micronuclei in each test group and the controls. It also comprises the usage of cell lineages of transgenic non-human animals with fluorescence-labelled nuclei for the *in vivo* and/or *ex-vivo* scoring of MN after exposure to samples / aqueous solutions.

The invention covers also test kits suitable for the detection of the genotoxic potential of samples by the presented method. One test kit comprises at least one vial with cryo-conserved transgenic stem cells from a non-human animal with nuclei labelled with a fluorescent protein fused to a chromatin-associated protein, at least one cell culture plate to be prepared and pre-cultured with the stem cell line for a couple of days in the customary procedure, lyophilised cell-culture media to be rehydrated with aqueous solutions in different concentrations of the test compounds, and positive control agents.

Another test kit comprises cryo-conserved transgenic stem cells from a non-human animal with nuclei labelled with fluorescent protein fused to a chromatin-associated protein conserved as a monolayer on at least one cell coated culture well plate ready to use after thawing for micronucleus testing, lyophilised cell-culture media to be rehydrated with aqueous solutions in different concentrations of the test compounds, and positive control agents.

Preliminary results with the presented invention show that the inventive cell line is suitable for the MNvit assay, enabling visual scoring of normal cell nuclei, fragmented nuclei and MN by fluorescence microscopy using a Motic AE 21 microscope with special filters for fluorescence microscopy, a Zeiss Axiocam MRc 5 camera and a LQ-HXP 120 (LEJ) compact light source.

The inventive cells were cryopreserved in a solution consisting of 10 % DMSO and 10 % FBS (fetal-bovine-serum) in MEM (minimum-essential-medium) at -80 °C. The cells were thawed, washed with MEM and transferred to a 96 micro-well plate. In order to avoid evaporation of medium and consequent bias in MN frequency, the outer wells were left empty and the micro-well plate was sealed with duct tape. The inner wells were filled with cells in medium. The medium employed was composed of 86 % MEM, 10 % FBS, 1 % non-essential-amino acids, 1 % streptomycin (5 mg/ml in PBS -phosphate-buffered saline), 1 % penicillin (3.125 mg/ml in PBS) and 1 % amphotericin (250 µg/ml in deionized water). The cells were cultivated at 26 °C until they formed a moderately dense monolayer. The time until optimal monolayer density was achieved varied from two to five days.
8 rows with cells were then exposed to colchicine (aneugen) or to 4NQO (4-Nitroquinoline 1-oxide, clastogen) as positive controls for 12 to 14 hours in a temperature-controlled environment (26° - 28° Celsius).

Under real conditions in routine applications, some rows with cells will then additionally be exposed to aquatic samples and/or aqueous solutions of potentially genotoxic test compounds in different concentrations. These aqueous solutions can also be used to rehydrate lyophilised cell-culture media (in an instant form as part of a test kit) before being pipetted.

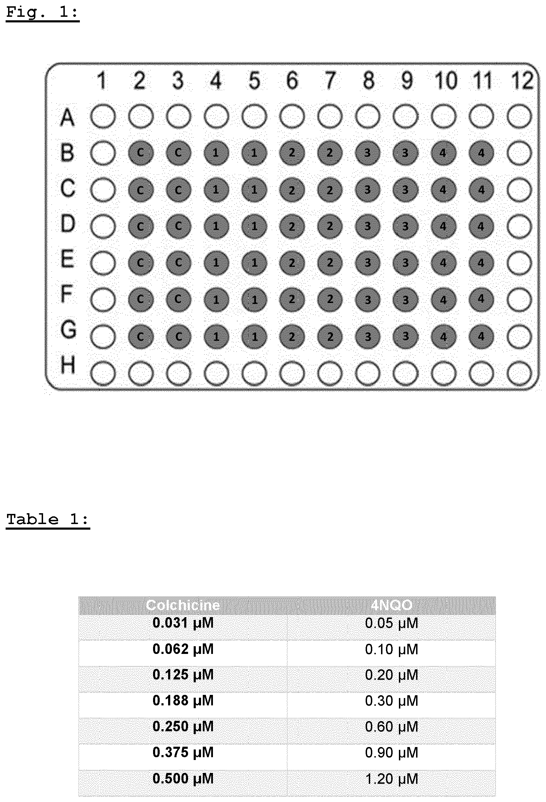

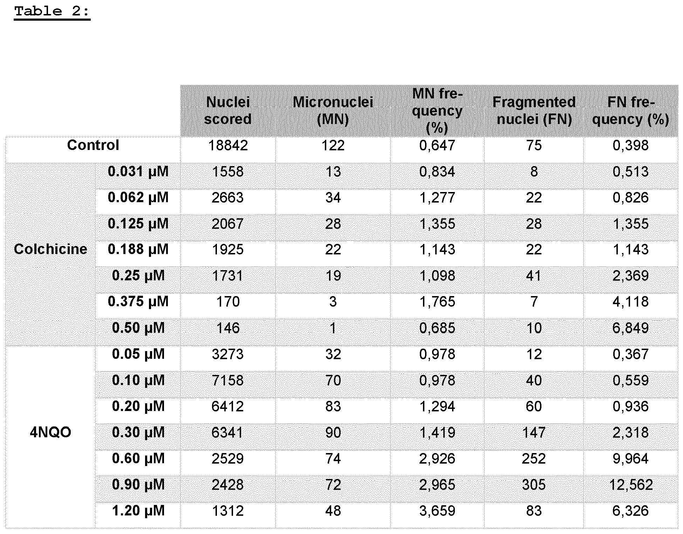

In the basic test, four different concentrations of each chemical agent (colchicine and 4NQO) were used in each plate, each concentration in two columns, and a total of 7 concentrations (including amounts above the cytotoxic threshold) were tested for validating the assay. Two untreated (i.e. not exposed to genotoxic compounds) columns served as internal (negative) controls of each plate. The pipetting scheme used is shown in figure 1.

These experiments were performed six times for each chemical agent.

Figure 1. Pipetting scheme used for the 96 micro-well plate. C (control) stands for wells with untreated (non-exposed) KCB cells and 1 to 4 for cells exposed to different concentrations of colchicine or 4-NQO.

The concentrations of colchicine and 4NQO employed for these experiments are displayed in table 1, with 4 concentrations per plate, following the scheme presented in figure 1.

Table 1. Concentrations of colchicine and 4NQO employed in the validation of the method for MN frequency assessment.

It is possible, but not absolutely necessary, to replace the exposure medium by a recovery medium before the scoring. The scoring of micronuclei shall take place earliest 2 hours and latest 12 hours after the end of the exposure period.

In the basic test, a minimum of two pictures per well, corresponding to at least 24 pictures per concentration or control, were taken under a fluorescence microscope with an objective magnification of 40x. Normal cell nuclei, MN and fragmented nuclei in each picture were counted visually with aid of the "Cell Counter" plugin from the public domain image processing software "ImageJ". Finally, the frequencies of MN per concentration were calculated.

To enable the detection of nucleoplasmic bridges and single chromosomes, a different microscope and camera with a higher resolution were used. For this, cover slips were placed in a 12 micro-well plate where the thawed cells were cultured. The cells were left to grow and proliferate on top of the cover slips at 26 °C for one or two days until they formed a moderately dense monolayer. The medium was removed and the cells were fixed by adding 1 ml of a 4 % solution of PFA (Paraformaldehyde) for 20 to 30 minutes at 4°C. Cover slips were then mounted on slides with Roti-Mount FluorCare (Carl Roth). Subsequently, the slides were stored at 4°C until photographs were taken of normal nuclei, MN, fragmented nuclei and nucleoplasmic bridges by using a Zeiss Axioskop 40 with an objective magnification of 100x. A high resolution camera additionally magnified (digitally) the cell/ nuclei 10 times.

Given the significantly better representation of the nuclear morphology when using a transient microscope, it seems to be promising to test the visualization of micronuclei of isolated cells after trypsinization and transfer to standard microscope slights

### Results

The detection of MN and a significant, dose-dependent increase in MN frequency could be verified in KCB cells labelled with the H2B-GFP fusion protein by using fluorescence microscopy.

Genotoxic potential assessment of the tested substances (colchicine and 4NQO) was performed by visually scoring MN and calculating their frequency in relation to the total number of nuclei scored. The counting results for the experiments are summed up in table 2.

Table 2. Total number of micronuclei and fragmented nuclei, as well as their frequencies (relative to the total number of assessed cells /nuclei) in the negative controls and in 12-14 hours exposure of cells to different concentrations of colchicine and 4NQO.

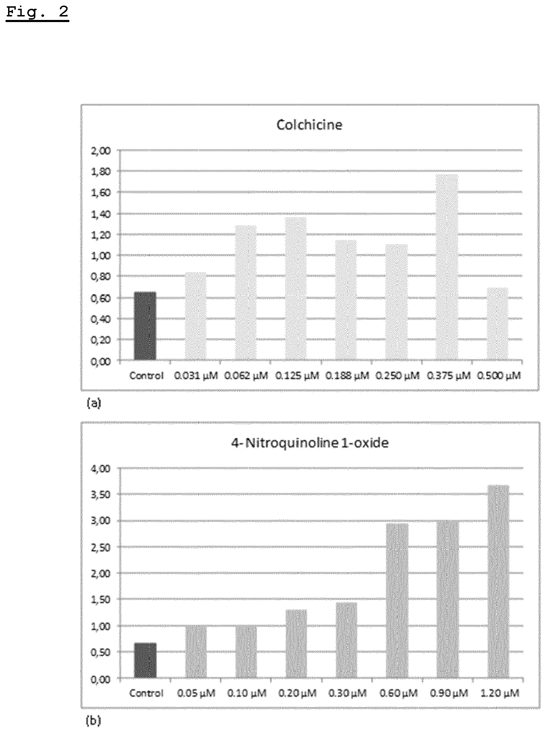

Cells exposed to colchicine and 4NQO clearly presented higher MN frequencies than the control (table 2, figure 2. a, b), and also higher frequencies of fragmented nuclei (table 2). Exposure to 4NQO induced higher maximal MN frequency (3.66 % at 1.2 µM) than colchicine (1.77 % at 0.375 µM), and a higher maximal frequency of fragmented nuclei (12.56 % at 0.9 µM) than colchicine (6.85 % at 0.5 µM).

Figure 2. MN frequency after exposure to different concentrations of (a) colchicine and (b) 4NQO.

In the experiments with 4NQO, both the MN frequency and the frequency of fragmented nuclei rise with increasing concentrations. After the exposure to colchicine, overall MN frequency has risen in a dose-dependent manner up to 0.125 µM, where a shift in MN frequency shows decreasing values with increased concentrations. Although this pattern is interrupted by the high MN frequency of the 0.375 µM group, the MN frequency further decreases for 0.5 µM, and both results must be considered of little significance due to the small numbers of cells/nuclei assessed (see table 2).

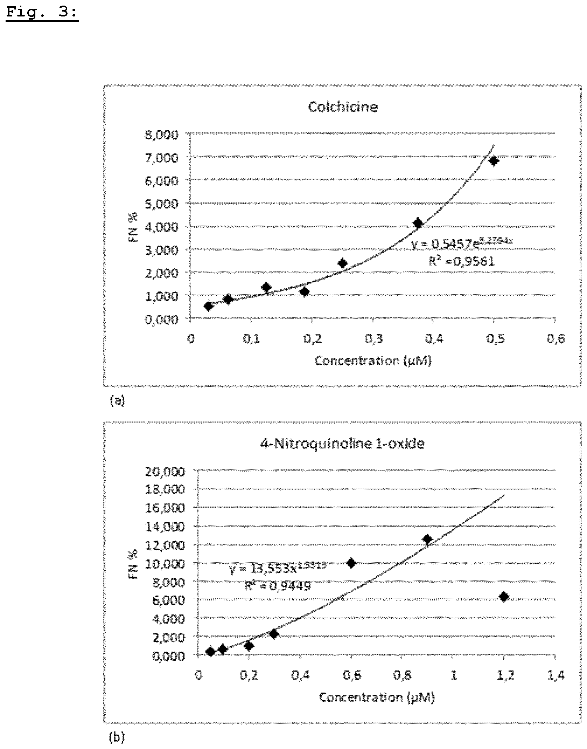

The frequency of fragmented nuclei seems to present a geometric progression with increased concentrations, at least up to a certain point (which seems to be close to 0.5 µM for colchicine and between 0.6 and 0.9 µM for 4NQO), as evidenced by the correlation between concentrations and fragmented nuclei frequencies in both cases (figure 3. a, b).

Figure 3. Frequency of fragmented nuclei in relation to different concentrations of (a) colchicine and (b) 4NQO.

The coefficients of determination (R²) close to 95 % indicate a very good fit of the data to the exponential trend line displayed in figures 3 a and b, although in the case of 4NQO (fig. 3. b) the frequency of fragmented nuclei for the 1.2 µM group had to be left out for the analysis to be meaningful. In any case, FN frequencies in both cases show a dose-response relationship to the concentration of these substances.

### Discussion

Cells not treated with a chemical agent (controls) clearly show a lower MN frequency than cells treated with either colchicine or 4NQO. The MN frequency initially rises as the concentration of the genotoxic substance is increased (table 2; fig. 2), and for colchicine it decreases in concentrations higher than 0.188 µM group (with the exception of the 0.375 µM group). For 4NQO, on the other hand, no decrease in higher concentration ranges has been observed, although a dose-dependent increase for concentrations higher than 0.6 µM could not be observed. These results indicate that a cytotoxic threshold may have been achieved at these concentrations, which in the case of colchicine leads to a higher mortality rate in affected cells (hence the diminishing MN frequency in concentrations higher than 0.188 µM, fig. 2. a). The stabilization of MN frequency and increase in FN rates in 4NQO indicate that the higher concentrations (especially in the range of 0.6 µM and higher) induced both cytotoxicity and genotoxicity, which would explain the relative stabilization of MN frequency for 4NQO in this range (fig. 2. b).

Surrales and collaborators (1994) have observed a MN frequency increase from 1% (in controls) to around 15.2% in human lymphocytes exposed for 24 hours to colchicine at 0.06 µM. Lofti & Santelli (2006) observed MN frequencies up to 4.7 % after 72 hours exposure to colchicine at 20 ng/mL (around 0.05 µM) in human skin fibroblasts, which background frequencies range between 0.2 and 0.9 %. In terms of *in vivo* studies, MN frequency in carp (*Cyprinus carpio*) erythrocytes increased from a background of 2% to around 10% in fish injected with 0.4 mg colchicine / kg body weight (Gustavino et al 2001). In erythrocytes of the European minnow (*Phoxinus phoxinus*) and of the sailfin molly (*Poecilia latipinna*), MN frequency increased from a background of 6.25 to 20.76% in fish injected with 10 mg colchicine / kg body weight (Aylon & Garcia-Vasquez 2000).

As for 4NQO, Valentin-Severin et al (2003) describe how the MN frequency increases from a background of 1% up to 3.5% (at 2 µM) in the human liver carcinoma cell line HepG2. In the mouse lymphoma cell line L5178Y, MN frequency increases from around 0.2% (controls) to around 1.8% after 4 hours exposure to 4NQO at approximately 0.25 µM (Brüsehafer et al 2015). *In vivo* results with zebrafish (*Danio rerio*) erythrocytes show an increase in MN frequency from a background around 0.3% to values between 1% and 1.7% when exposed to 4NQO at 2.9 µg/L in the water with a flow-through system (Diekmann et al 2004).

These results show a maximum 20-fold increase in MN frequency in cell lines exposed to colchicine and around 5-fold increase in individuals injected with the aneugen. Exposure of cell lines to 4NQO leads to a maximal 10-fold increase in MN frequency in the cell lines described, and around 6-fold increase in the erythrocytes of fish exposed to the clastogen. In general terms, these results are significantly higher than the 3- (for colchicine) and 4-fold (for 4NQO) increase observed in the cell line presented herein, but are not directly comparable considering the higher concentrations and/or longer exposure periods employed in the literature data.

The present inventive method simplifies the MNvit assay by avoiding both the harvesting and staining steps involved in alternative methods, allowing for visual and/or automated scoring (through image analysis software) of MN frequency in an easy to handle and low-maintenance cell line. The fluorescence labelled nuclei permit visualization of micronuclei through simple fluorescence microscopy, without need of high-end devices (e.g. flow cytometer) and or expert personnel, and allow for the monitoring of living cells after MN scoring for further information regarding genotoxic effects.

The presented inventive method is also suitable to distinguish between unaffected cells and carcinogenic transformed cells for direct reflection of the carcinogenic potential of the test compounds by using a differential fluorescent measurement after the non-destructive scoring process.

The cited state of the art is presented in the following list of documents:
Aylon F., Garcia-Vasquez, E. 2000. Induction of micronuclei and other nuclear abnormalities in European minnow Phoxinus phoxinus and mollie Poecilia latipinna: an assessment of the fish micronucleus test. Mutat Res., 467(2):177-86.
Brüsehafer K., Manshian B. B., Doherty A. T., Zai'r Z. M., Johnson G. E., Doak S. H., Jenkins G. J., 2015. The clastogenicity of 4NQO is cell-type dependent and linked to cytotoxicity, length of exposure and p53 proficiency. Mutagenesis pii: gev069. [Epub ahead of print]
Gustavino B., Scornajenghi K. A., Minissi S., Ciccotti E., 2001. Micronuclei induced in erythrocytes of Cyprinus carpio (teleostei, pisces) by X-rays and colchicine. Mutat Res., 494 (l-2) :151-9.
Lotfi C. F., Machado-Santelli G. M., 1996. Comparative analysis of colchicine induced micronuclei in different cell types in vitro. Mutat Res., 349(l):77-83.
Surralles J., Antoccia A., Creus A., Degrassi F., Peris F., Tanzarella C, Xamena N., Marcos R., 1994. The effect of cyto-chalasin-B concentration on the frequency of micronuclei induced by four Standard mutagens. Results from two laboratories. Mutagenesis, 9(4):347-53.
Valentin-Severin I., Le Hegarat L, Lhuguenot J. C, Le Bon A. M., Chagnon M. C, 2003. Use of HepG2 cell line for direct or indirect mutagens Screening: comparative investigation between comet and micronucleus assays. Mutat Res, 536(l-2):79-90.

## Claims

1. Transgenic indicator stem cell line of a non-human animal for non-destructive, self-signalizing visualization of nuclear structures, which allows the identification of nuclear and chromosome anomalies - including micronuclei - as consequence of exposure to genotoxic compounds wherein
- the stem cell line is a stem cell line with nuclei labelled with the green fluorescent protein (GFP)fused to the chromatin-associated protein histone 2B, and wherein
- the transgenic stem cells are pluripotent and tissue-regenerating.

2. The transgenic indicator stem cell line according to claim 1, wherein
said transgenic stem cell line is from a vertebrate.

3. The transgenic indicator stem cell line according to claim 2, wherein
said transgenic stem cell line is from fish.

4. The transgenic indicator stem cell line according to claim 3, wherein
said transgenic stem cell line is from Koi carp (Cyprinus carpio haematopterus).

5. The transgenic indicator stem cell line according to any one of the claims 1 to 4, wherein
said transgenic stem cell line is from the non-human animal brain.

6. The transgenic indicator stem cell line according to any one of the claims 1 to 5,
which is deposited, in accordance with the Budapest Treaty, at the "Deutsche Sammlung von Mikroorganismen und Zellkulturen (DSMZ)" under the number DSM ACC3285.

7. Transgenic non-human living organism for non-destructive, self-signalizing visualization of nuclear structures, which allows the identification of nuclear and chromosomal anomalies - including micronuclei - as consequence of exposure to genotoxic compounds,
- in which a cell lineage has nuclei labelled with the green fluorescent protein (GFP) fused to the chromatin-associated protein histone 2B in the labelled nuclei, and wherein
- the indicator transgenic non-human living organism has a cell lineage such as germ cells or erythroid cells showing a lineage specific expression of the histone fused fluorescent protein, and wherein
- the transgenic non-human living indicator organism is a fish.

8. Transgenic non-human living organism according to claim 7,
wherein
the fish is Medaka (Oryzias latipes).

9. Use of a transgenic non-human living organism according to claim 7, wherein
the MN visualization and scoring is conducted in blood cells of living translucent embryos or ex vivo by preparation of blood smears.

10. A method for the detection of the genotoxic potential of aquatic samples or aqueous solutions of test compounds by using a transgenic indicator stem cell line of claim 1, the method comprising:
- the use of a transgenic stem cell line from a non-human animal with nuclei labelled with the green fluorescent protein (GFP) fused to the chromatin-associated protein histone 2B growing as a monolayer on at least one cell culture plate
- and cell-culture media supplemented with different concentrations of the test compounds,
- pipetting these media to the exposure wells on the at least one cell coated culture plate and
- comparing micronucleus frequency with positive and negative controls after cell division by non-destructive visual scoring micronuclei in each test group and the controls.

11. The method of claim 10, wherein
there is an exposure period after the pipetting of the dilution media to the exposure wells on the at least one cell coated culture plate, preferably about 6 - 16 hours.

12. The method according to any one of the claims 10 to 11,
wherein
the exposure takes place in a temperature-controlled environment, preferably between 26° - 28° Celsius.

13. The method according to any one of the claims 10 to 12,
wherein
the exposure medium is replaced by a recovery medium before the scoring.

14. The method according to any one of claims 10 to 13, wherein the scoring of micronuclei shall take place earliest 2 hours and latest 12 hours after the end of the exposure period.

15. The method according to any one of the claims 10 to 14,
wherein
after the non-destructive scoring process a differential fluorescent measurement is used to distinguish between unaffected cells and carcinogenic transformed cells for direct reflection of the carcinogenic potential of the test compound.

16. The method according to any one of the claims 10 to 15,
wherein
the cell-culture media are lyophilised and rehydrated
with aqueous solutions in different concentrations of the test compounds.

17. The method according to any one of the claims 10 to 16,
wherein
the transgenic stem cell line from cells of a non-human animal with nuclei labelled with the green
fluorescent protein (GFP) fused to the chromatin-associated protein histone 2B is the stem cell line, which
is deposited, in accordance with the Budapest Treaty, at the "Deutsche Sammlung von Mikroorganismen und Zellkulturen (DSMZ)" under the number DSM ACC3285.

18. A kit suitable for the detection of the genotoxic potential of samples by the method according to any one of the claims 10 to 17, which comprises:
- at least one vial with a cryo-conserved transgenic stem cell line from a non-human animal with nuclei labelled with the green fluorescent protein (GFP) fused to the chromatin-associated protein histone 2B,
- at least one cell culture plate to be prepared and pre-cultured with the stem cell line for a couple of days in the customary procedure,
- lyophilised cell-culture media to be rehydrated with aqueous solutions in different concentrations of the test compounds
- and positive control agents.

19. A kit suitable for the detection of the genotoxic potential of samples by the method according to any one of the claims 10 to 17, which comprises:
- a cryo-conserved transgenic stem cell line from a non-human animal with labelled nuclei with the green fluorescent protein (GFP) fused to the chromatin-associated protein histone 2B
conserved
as a monolayer of vital cells on at least one cell coated culture plate ready to use after thawing for micronucleus testing
- and lyophilised cell culture media to be rehydrated with aqueous solutions in different concentrations of the test compounds
- and positive control agents.

## Patentansprüche

1. Transgene Indikator-Stammzelllinie eines nichtmenschlichen Tiers zur nichtdestruktiven, selbstsignalisierenden Visualisierung von Zellkernstrukturen, was die Identifizierung von Kern- und Chromosomenanomalien - einschließlich Mikronuklei - als Folge eines In-Kontakt-Kommens mit genotoxischen Verbindungen gestattet, wobei
- es sich bei der Stammzelllinie um eine Stammzelllinie mit Zellkernen, die mit dem an das chromatinassoziierte Protein Histon 2B fusionierten grünen Fluoreszenzprotein (GFP) markiert sind, handelt und wobei
- die transgenen Stammzellen pluripotent und geweberegenerierend sind.

2. Transgene Indikator-Stammzelllinie nach Anspruch 1, wobei
die transgene Stammzelllinie von einem Vertebraten stammt.

3. Transgene Indikator-Stammzelllinie nach Anspruch 2, wobei
die transgene Stammzelllinie von Fischen stammt.

4. Transgene Indikator-Stammzelllinie nach Anspruch 3, wobei
die transgene Stammzelllinie von Koi-Karpfen (Cyprinus carpio haematopterus) stammt.

5. Transgene Indikator-Stammzelllinie nach einem der Ansprüche 1 bis 4, wobei
die transgene Stammzelllinie vom Gehirn des nichtmenschlichen Tiers stammt.

6. Transgene Indikator-Stammzelllinie nach einem der Ansprüche 1 bis 5,
die gemäß dem Budapester Vertrag bei der DSMZ (Deutsche Sammlung von Mikroorganismen und Zellkulturen) unter der Nummer DSM ACC3285 hinterlegt ist.

7. Transgener nichtmenschlicher Lebendorganismus zur nichtdestruktiven, selbstsignalisierenden Visualisierung von Zellkernstrukturen, was die Identifizierung von Kern- und Chromosomenanomalien - einschließlich Mikronuklei - als Folge eines In-Kontakt-Kommens mit genotoxischen Verbindungen gestattet,
- bei dem eine Zellabstammungslinie Zellkerne aufweist, die mit dem grünen Fluoreszenzprotein (GFP) markiert sind, das an das chromatinassoziierte Protein Histon 2B in den markierten Zellkernen fusioniert ist, und wobei
- der indikatortransgene nichtmenschliche Lebendorganismus eine Zellabstammungslinie, wie z. B. Keimzellen oder erythroide Zellen, aufweist, die eine abstammungsspezifische Expression des histonfusionierten Fluoreszenzproteins zeigt, und wobei
- es sich bei dem transgenen nichtmenschlichen Indikator-Lebendorganismus um einen Fisch handelt.

8. Transgener nichtmenschlicher Lebendorganismus nach Anspruch 7, wobei
es sich bei dem Fisch um Medaka (Oryzias latipes) handelt.

9. Verwendung eines transgenen nichtmenschlichen Lebendorganismus nach Anspruch 7, wobei
die MN-Visualisierung und -Zählung (Scoring) in Blutzellen lebender transluzenter Embryos oder ex vivo durch Präparation von Blutausstrichen erfolgt.

10. Verfahren zum Nachweis des genotoxischen Potentials aquatischer Proben oder wässriger Lösungen von Testverbindungen unter Verwendung einer transgenen Indikator-Stammzelllinie gemäß Anspruch 1, wobei das Verfahren Folgendes umfasst:
- die Verwendung einer transgenen Stammzelllinie aus einem nichtmenschlichen Tier mit mit dem an das chromatinassoziierte Protein Histon 2B fusionierten grünen Fluoreszenzprotein (GFP) markierten Zellkernen, die als Einzelschicht (Monolayer) auf wenigstens einer Zellkulturplatte wächst,
- und Zellkulturmedien supplementiert mit unterschiedlichen Konzentrationen der Testverbindungen,
- Pipettieren dieser Medien in die Kontakt-Wells auf der wenigstens einen zellbeschichteten Kulturplatte und
- Vergleichen der Mikronukleihäufigkeit mit Positiv- und Negativkontrollen nach Zellteilung durch nichtdestruktives visuelles Zählen von Mikronuklei in jeder Testgruppe und den Kontrollen.

11. Verfahren nach Anspruch 10, wobei
es nach dem Pipettieren der Verdünnungsmedien in die Kontakt-Wells auf der wenigstens einen zellbeschichteten Kulturplatte einen Kontaktzeitraum gibt, vorzugsweise etwa 6 - 16 Stunden.

12. Verfahren nach einem der Ansprüche 10 bis 11, wobei
der Kontakt in einer temperaturkontrollierten Umgebung, vorzugsweise zwischen 26° - 28° Celsius, stattfindet.

13. Verfahren nach einem der Ansprüche 10 bis 12, wobei
das Kontaktmedium vor dem Zählen durch ein Erholungsmedium ersetzt wird.

14. Verfahren nach einem der Ansprüche 10 bis 13, wobei das Zählen der Mikronuklei frühestens 2 Stunden and spätestens 12 Stunden nach dem Ende des Kontaktzeitraums stattfindet.

15. Verfahren nach einem der Ansprüche 10 bis 14, wobei
nach dem nichtdestruktiven Zählvorgang eine differentielle Fluoreszenzmessung zur Unterscheidung zwischen nicht betroffenen Zellen und karzinogenen transformierten Zellen als direktes Spiegelbild des karzinogenen Potentials der Testverbindung verwendet wird.

16. Verfahren nach einem der Ansprüche 10 bis 15, wobei
die Zellkulturmedien lyophilisiert und mit wässrigen Lösungen in unterschiedlichen Konzentrationen der Testverbindungen rehydratisiert werden.

17. Verfahren nach einem der Ansprüche 10 bis 16, wobei
es sich bei der transgenen Stammzelllinie aus Zellen eines nichtmenschlichen Tiers mit mit dem an das chromatinassoziierte Protein Histon 2B fusionierten grünen Fluoreszenzprotein (GFP) markierten Zellkernen um die Stammzelllinie handelt, die gemäß dem Budapester Vertrag bei der DSMZ (Deutsche Sammlung von Mikroorganismen und Zellkulturen) unter der Nummer DSM ACC3285 hinterlegt ist.

18. Kit, geeignet für den Nachweis des genotoxischen Potentials von Proben mit dem Verfahren nach einem der Ansprüche 10 bis 17, das Folgendes umfasst:
- wenigstens ein Fläschchen mit einer kryokonservierten transgenen Stammzelllinie von einem nichtmenschlichen Tier mit mit dem an das chromatinassoziierte Protein Histon 2B fusionierten grünen Fluoreszenzprotein (GFP) markierten Zellkernen,
- wenigstens eine Zellkulturplatte, die mit der Stammzelllinie ein paar Tage in herkömmlicher Vorgehensweise präpariert und vorkultiviert wird,
- lyophilisierte Zellkulturmedien, die mit wässrigen Lösungen in unterschiedlichen Konzentrationen der Testverbindungen rehydratisiert werden,
- und Positivkontrollagentien.

19. Kit, geeignet für den Nachweis des genotoxischen Potentials von Proben mit dem Verfahren nach einem der Ansprüche 10 bis 17, das Folgendes umfasst:
- eine kryokonservierte transgene Stammzelllinie von einem nichtmenschlichen Tier mit mit dem an das chromatinassoziierte Protein Histon 2B fusionierten grünen Fluoreszenzprotein (GFP) markierten Zellkernen, die als Einzelschicht vitaler Zellen auf wenigstens einer zellbeschichteten Kulturplatte konserviert und nach Auftauen gebrauchsfertig für Mikronukleustests ist,
- und lyophilisierte Zellkulturmedien, die mit wässrigen Lösungen in unterschiedlichen Konzentrationen der Testverbindungen rehydratisiert werden,
- und Positivkontrollagentien.

## Revendications

1. Lignée de cellules souches indicatrices transgéniques d'un animal non humain destinée à la visualisation non destructive, auto-signalisante de structures nucléaires, qui permet l'identification d'anomalies nucléaires et chromosomiques - y compris des micronoyaux - comme conséquence d'une exposition à des composés génotoxiques
- la lignée de cellules souches étant une lignée de cellules souches avec des noyaux marqués à la protéine fluorescente verte (GFP) fusionnée avec la protéine histone 2B associée à la chromatine et dans laquelle
- les cellules souches transgéniques sont pluripotentes et régénératrices de tissus.

2. Lignée de cellules souches indicatrices transgéniques selon la revendication 1,
ladite lignée de cellules souches transgéniques provenant d'un vertébré.

3. Lignée de cellules souches indicatrices transgéniques selon la revendication 2,
ladite lignée de cellules souches transgéniques provenant d'un poisson.

4. Lignée de cellules souches indicatrices transgéniques selon la revendication 3,
ladite lignée de cellules souches transgéniques provenant de la carpe Koï (Cyprinus carpio haematopterus).

5. Lignée de cellules souches indicatrices transgéniques selon l'une quelconque des revendications 1 à 4,
ladite lignée de cellules souches transgéniques provenant du cerveau d'un animal non humain.

6. Lignée de cellules souches indicatrices transgéniques selon l'une quelconque des revendications 1 à 5,
qui est déposée, selon le Traité de Budapest, au "Deutsche Sammlung von Mikroorganismen und Zellkulturen (DSMZ)" sous le numéro DSM ACC3285.

7. Organisme vivant non humain transgénique destiné à une visualisation non destructive, auto-signalisante de structures nucléaires, qui permet l'identification d'anomalies nucléaires et chromosomiques - y compris des micronoyaux - comme conséquence d'une exposition à des composés génotoxiques,
- dans lequel une lignée de cellules a des noyaux marqués à la protéine fluorescente verte (GFP) fusionnée avec la protéine histone 2B associée à la chromatine dans les noyaux marqués et
- l'organisme vivant non humain transgénique indicateur ayant une lignée de cellules, telle que de cellules germinales ou de cellules érythroïdes, présentant une expression, spécifique d'une lignée, de la protéine fluorescente fusionnée avec une histone et
- l'organisme indicateur vivant non humain transgénique étant un poisson.

8. Organisme vivant non humain transgénique selon la revendication 7, dans lequel
le poisson est un Médaka (Oryzias latipes).

9. Utilisation d'un organisme vivant non humain transgénique selon la revendication 7, dans lequel la visualisation MN et la notation sont conduites dans des cellules sanguines d'embryons translucides vivants ou ex vivo via une préparation de frottis sanguins.

10. Procédé destiné à la détection du potentiel génotoxique d'échantillons aquatiques ou de solutions aqueuses de composés à tester en utilisant une lignée de cellules souches indicatrices transgéniques de la revendication 1, le procédé comprenant :
- l'utilisation d'une lignée de cellules souches transgéniques provenant d'un animal non humain, avec des noyaux marqués à la protéine fluorescente verte (GFP) fusionnée avec la protéine histone 2B associée à la chromatine, se développant sous forme de monocouche sur au moins une plaque de culture cellulaire
- et des milieux de culture cellulaire enrichis en différentes concentrations des composés à tester,
- un pipetage de ces milieux vers les puits d'exposition sur l'au moins une plaque de culture enduite de cellules et
- une comparaison de la fréquence d'un micronoyau avec des témoins positifs et négatifs après une division cellulaire via une notation visuelle non destructive des micronoyaux dans chaque groupe de test et les témoins.

11. Procédé de la revendication 10, dans lequel après le pipetage des milieux de dilution, il y a une période d'exposition aux puits d'exposition sur l'au moins une plaque de culture enduite de cellules, de préférence de 6 à 16 heures environ.

12. Procédé selon l'une quelconque des revendications 10 à 11, dans lequel
l'exposition a lieu dans un environnement à température contrôlée, de préférence entre 26° et 28° Celsius.

13. Procédé selon l'une quelconque des revendications 10 à 12, dans lequel
le milieu d'exposition est remplacé par un milieu de récupération avant la notation.

14. Procédé selon l'une quelconque des revendications 10 à 13, dans lequel la notation des micronoyaux devra avoir lieu au plus tôt 2 heures et au plus tard 12 heures après la fin de la période d'exposition.

15. Procédé selon l'une quelconque des revendications 10 à 14, dans lequel
après le processus non destructeur de notation, une mesure de fluorescence différentielle est utilisée pour faire une distinction entre les cellules non affectées et les cellules carcinogènes transformées pour refléter directement le potentiel carcinogène du composé à tester.

16. Procédé selon l'une quelconque des revendications 10 à 15, dans lequel
les milieux de culture cellulaire sont lyophilisés et réhydratés avec des solutions aqueuses à différentes concentrations des composés à tester.

17. Procédé selon l'une quelconque des revendications 10 à 16, dans lequel
la lignée de cellules souches transgéniques provenant de cellules d'un animal non humain, avec des noyaux marqués à la protéine fluorescente verte (GFP) fusionnée avec la protéine histone 2B associée à la chromatine, est la lignée de cellules souches qui est déposée, selon le Traité de Budapest, au "Deutsche Sammlung von Mikroorganismen und Zellkulturen (DSMZ)" sous le numéro DSM ACC3285.

18. Trousse appropriée pour la détection du potentiel génotoxique d'échantillons par le procédé selon l'une quelconque des revendications 10 à 17, qui comprend :
- au moins un flacon avec une lignée cryo-conservée de cellules souches transgéniques provenant d'un animal non humain, avec des noyaux marqués à la protéine fluorescente verte (GFP) fusionnée avec la protéine histone 2B associée à la chromatine,
- au moins une plaque de culture cellulaire devant être préparée et pré-cultivée avec la lignée de cellules souches pendant environ deux jours dans la procédure habituelle,
- des milieux de culture cellulaire lyophilisés devant être réhydratés avec des solutions aqueuses à différentes concentrations des composés à tester
- et des agents témoins positifs.

19. Trousse appropriée pour la détection du potentiel génotoxique d'échantillons par le procédé selon l'une quelconque des revendications 10 à 17, qui comprend :
- une lignée cryo-conservée de cellules souches transgéniques provenant d'un animal non humain, avec des noyaux marqués à la protéine fluorescente verte (GFP) fusionnée avec la protéine histone 2B associée à la chromatine, conservée sous forme de monocouche de cellules vitales sur au moins une plaque de culture enduite de cellules prête à l'emploi après décongélation en vue d'un test de micronoyau
- et des milieux de culture cellulaire lyophilisés devant être réhydratés avec des solutions aqueuses à différentes concentrations des composés à tester
- et des agents témoins positifs.
